Europäisches Patentamt

European Patent Office

Office européen des brevets

(19)

(11) **EP 0 571 387 B1**

(12) **EUROPEAN PATENT SPECIFICATION**

(45) Date of publication and mention
of the grant of the patent:
**17.12.2003 Bulletin 2003/51**

(51) Int Cl.⁷: **C12N 15/12**, C07K 14/475,
A61K 38/18, C12N 5/10

(21) Application number: **91917603.2**

(22) Date of filing: **10.09.1991**

(86) International application number:
**PCT/US91/06368**

(87) International publication number:
**WO 9200/5184 (02.04.1992 Gazette 1992/08)**

(54) **A NON-MITOGENIC COMPETITIVE HGF ANTAGONIST**

EIN NICHT-MITOGENER KOMPETITIVER HGF-ANTAGONIST

ANTAGONISTE DE FACTEUR DE CROISSANCE D'HEPATOCYTES COMPETITIF NON
MITOGENE

(84) Designated Contracting States:
**AT BE CH DE DK ES FR GB GR IT LI LU NL SE**

(30) Priority: **14.09.1990 US 582063**
**15.02.1991 US 655502**

(43) Date of publication of application:
**01.12.1993 Bulletin 1993/48**

(73) Proprietor: **THE UNITED STATES OF AMERICA as
represented by the Secretary United States
Department of Commerce
Springfield, Virginia 22161 (US)**

(72) Inventors:
 • **RUBIN, Jeffrey, S.**
 **Rockville, MD 20853 (US)**
 • **CHAN, Andrew, M.-L.**
 **Rockville, MD 20852 (US)**
 • **AARONSON, Stuart, A.**
 **Great Falls, VA 22066 (US)**
 • **BOTTARO, Donald, P.**
 **Kensington, MD 20895 (US)**

(74) Representative: **Portal, Gérard et al
Cabinet Beau de Loménie
158, rue de l'Université
75340 Paris Cédex 07 (FR)**

(56) References cited:
 **EP-A- 0 246 753          WO-A-90/10651**

 • **NATURE. vol. 342 , 23 November 1989 , LONDON
 GB pages 440 - 443 T. NAKAMURA ET AL
 'Molecular cloning and expression of human
 hepatocyte growth factor'**
 • **BIOCHEMICAL AND BIOPHYSICAL RESEARCH
 COMMUNICATIONS. vol. 163, no. 2 , 15
 September 1989 , DULUTH, MINNESOTA US
 pages 967 - 973 K. MIYAZAWA ET AL 'Molecular
 cloning and sequence analysis of cDNA for
 human hepatocyte growth factor'**

**Description**

**Background of the Invention**

This is a continuation-in-part of the application serial number 07/582,063 filed September 14, 1990, the entire contents thereof being hereby incorporated by reference.

**Field of the Invention**

The present invention relates to a truncated form of hepatocyte growth factor (HGF), encoded by an alternative HGF mRNA transcript, which specifically antagonizes the mitogenic activity of HGF. In particular, the present invention relates to a small HGF variant which functions as a competitive antagonist at the level of HGF binding to its cell surface receptor.

The present invention further relates to methods of diagnosing and treating conditions in which cell proliferation is either excessive, as in the case of malignancy, or impaired, in part due to aberrant expression of the various forms of HGF.

**Background Information**

Hepatocyte growth factor has hormone-like activity and is released in response to partial hepatectomy and liver injury and is presumed to be an important mediator of liver regeneration (Nakamura et al., *Proc. Natl. Acad. Sci. U.S.A* 84: 6489-6493 (1986); Gohda et al., *J. Clin. Invest.* 81:414-419 (1988); R. Zarnegar and G. Michalopoulous *Cancer Research* **49**:3314-3320 (1989)). Its ubiquitous expression by stromal fibroblasts and demonstrated ability to stimulate DNA synthesis in melanocytes and endothelial cells as well as epithelial cells suggest that this factor plays a role in paracrine regulation of cell growth as well (Rubin et al. *Proc. Natl. Acad. Sci. USA* **88:** 415 (1991)). Recent reports of the purification of scatter factor, which shows high amino acid sequence identity to HGF over restricted regions, suggests that HGF may also be involved in modulating cell-cell interactions and migration (E. Gherardi and M. Stoker *Nature* **346**: 228 (1990) ; Weidner et al. *J. Cell Biology* 111:2097-2108 (1990)).

Structurally, HGF resembles plasminogen in that it possesses characteristic kringle domains (Patthy et al. *FEBS Lett* 171:131-136 (1984)) and a serine protease-like domain (Miyazawa et al. *Biochem. Biophys. Res. Commun.* **163**:967-973 (1989); Nakamura et al. *Nature* **342**:440-443 (1989)). Like plasminogen, HGF can be processed by proteolytic cleavage, generating a heterodimeric molecule comprised of a heavy- and light-chain covalently linked by disulfide bonds (Nakamura et al., *Proc. Natl. Acad. Sci. U.S.A.* **83**:6489-6493 (1986); Gohda et al. *J. Clin. Invest.* **81**: 414-419 (1988) ; Zarnegar et al. *Cancer Research* **49**: 3314-3320 (1989)). The possibility that its actions might be mediated by a receptor tyrosine kinase was suggested by its rapid stimulation of tyrosine phosphorylation of cellular proteins in target cells (Rubin et al., *Proc. Natl.Acad.Sci.USA* **88**:415 (1991)). Recent studies have directly identif ied the HGF receptor as the *c-met* protooncogene product (Bottaro et al., *Science* **25**1:802 (1991)), whose structure resembles that of a membrane-spanning tyrosine kinase (Park et al. *Proc. Natl. Acad. Sci. U.S.A* **84**:6379-6383 (1987) ; Chan et al. *Oncogene* **2**:593-599 (1988)).

There is accumulating evidence that the positive effects of growth factors on cell proliferation can be counteracted at a variety of levels both intracellularly (Moses et al. *Cell* **63**:245-247 (1990) and at the cell surface (Hannum et al., *Science* 343:336-340 (1990), Eisenberg, et al., *Nature* **343**:341-346 (1990); Carter et al., *Nature* **344**:633-637 (1990)). Thus, the potential exists to find an antagonist to HGF which would negatively regulate the growth factor's proliferative effects. The invention described herein relates to small HGF variants and their corresponding transcripts. Characterization of one of these HGF variants has revealed that it is a competitive antagonist of HGF action and thus establishes a novel regulatory mechanism whereby the same gene encodes both an agonist and antagonist of growth factor action.

Summary of the Invention

The invention is defined by the claims which are incorporated herein in their entirety by reference.

It is an object of the present invention to provide a specific inhibitor of hepatocyte growth factor (HGP) action identified as a smaller form of HGF encoded by an alternative HGF transcript which specifies a sequence that includes the N-terminal and first two kringle domains (HGF/NK2). This truncated HGF variant specifically antagonizes the mitogenic activity of HGF by competitively binding to the cell surface receptor for HGF. The variant itself lacks mitogenic activity.

In one embodiment, the present invention relates to the truncated HGF variant, HGF/NK2 which has an apparent molecular weight of 34 kd by SDS-PAGE under reducing conditions and is substantially free of proteins with which the variant is normally associated.

In another embodiment, the present invention relates to a DNA fragment encoding the 34 kilodalton HGF variant protein.

Another embodiment of the present invention relates to another small form of HGF besides the 34 kd variant that is encoded by an alternative HGF transcript which specifies a sequence that includes the N-terminal and only the first kringle domain (HGF/NK1).

In yet another embodiment, the present invention relates to a DNA fragment encoding HGF/NK1 with a predicted size of approximately 20 kilodaltons.

In a further embodiment, the present invention relates to a recombinant DNA molecule comprising a fragment of the above described DNA and a vector. The invention also relates to a host cell stably or transiently transformed with such a recombinant DNA molecule in a manner allowing expression of the small HGF variant protein encoded in the DNA fragment.

In another embodiment, the present invention relates to a method of producing a recombinant HGF truncated variant with HGF inhibitory activity which method comprises culturing host cells expressing HGF variant protein in a manner allowing expression of the protein and isolating the protein from the host cells.

In a further embodiment, the present invention relates to a method of producing HGF truncated variant protein in cultured cells substantially free of other proteins comprising the steps of culturing HGF variant producing cells in culture medium, contacting HGF variant culture medium with heparin affinity resin under conditions such that a complex between the variant and heparin is formed, separating the complex from the bulk of other protein in the medium, dissociating the HGF variant from the heparin affinity resin and finally fractionating the variant over a sizing column in order to separate any remaining contaminants from HGF variant.

The present invention also relates to cDNA clones that encode the truncated HGF variants, HGF/NK2 and HGF/NK1.

In a further embodiment, the present invention relates to therapeutic applications of the HGF inhibitor variant (s) in proliferative disorders including both cancer and non-malignant conditions in which HGF is excessive. The method comprises specif ically blocking the action of HGF by adminstering a therapeutic amount of HGF inhibitor to a clinical sample or by inducing the endogenous expression of increased amounts of the inhibitor.

The present invention permits implementation of therapeutic methods that decrease the overproduction of inhibitory HGF variant(s) that are inappropriately produced at high levels in a setting of impaired cell renewal. The method comprises specifically blocking the synthesis or action of the inhibitor HGF molecules by either contacting inhibitor HGF transcripts with antisense oligonucleotides or contacting inhibitor HGF protein with antibodies specific for the inhibitor molecules.

In yet another embodiment, the present invention permits implementation of methods of diagnosing pathological conditions in which cell growth is either impaired or excessive comprising the steps of isolating mRNA transcripts from a biological sample, contacting the mRNA transcripts with a DNA fragment encoding the inhibitory HGF variant, and detecting the presence of specif ic RNA-DNA hybrids to determine the level of inhibitory HGF variant expression in the sample. The method may also be performed by in situ hybridization in which the step of isolating mRHA transcripts from the sample is omitted before hybridization is carried out.

Various other objects and advantages of the present invention will become obvious from the drawings and the following description of the invention.

Brief Description of the Drawings

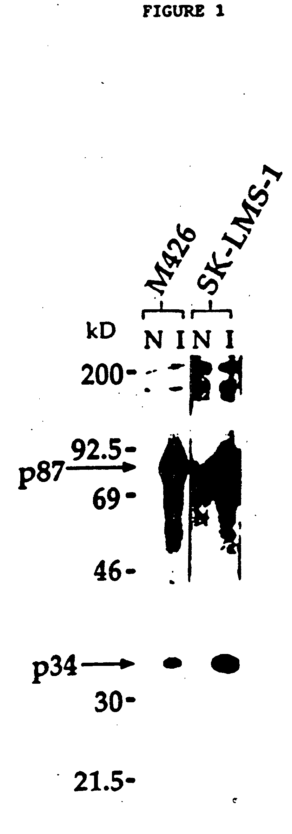

Figure 1 shows the detection of p34 in M426 and SK-LMS-1 cells. Equivalent amounts of ["S]-methionine and cysteine labeled conditioned medium from M426 and SK-LMS-1 cells were immunoprecipitated with nonimmune (N) and HGF immune-serum (I). Proteins were subjected to 10% SDS-PAGE under reducing conditions. HGFp87 and p34 are indicated by arrows, and molecular weight markers are shown in kD.

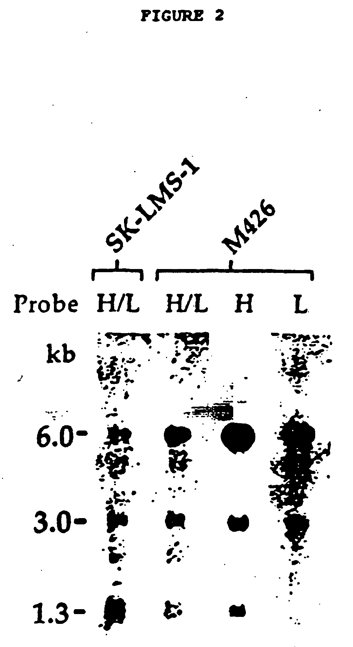

Figure 2 depicts the Northern analysis of RNA from M426 and SK-LMS-1 cells. Two µg of poly(A)$^\bullet$ RNA from SK-LMS-1 and M426 cells were electrophoresed on 1% agarose gels, and Northern blots were hybridized with either HGF coding region (H/L), heavy (H), or light (L) chain probes. The sizes in kilobases (kb) of three major HGF-related transcripts are indicated.

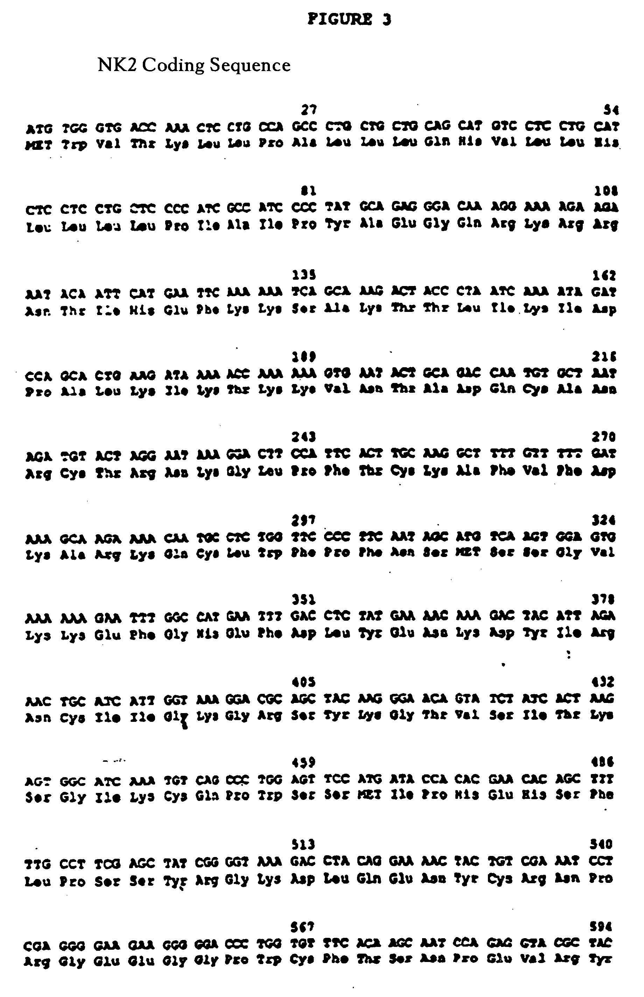

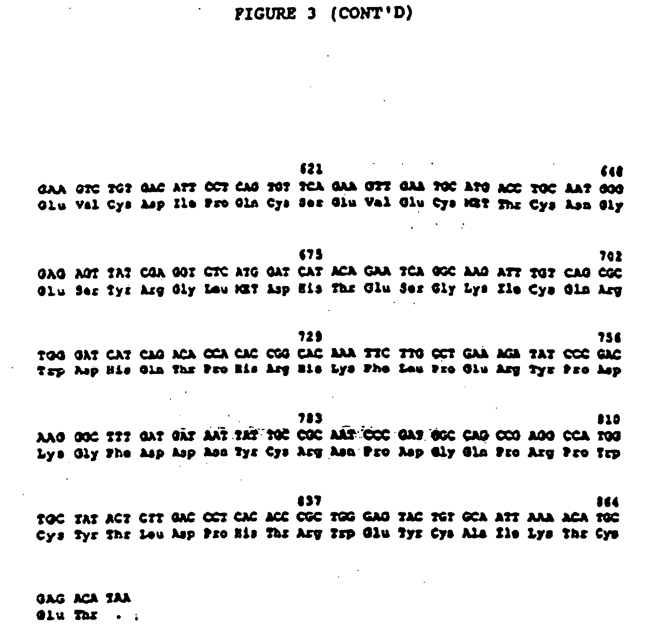

Figure 3, SEQ ID NO:1 and SEQ ID NO:2 show the cDNA coding sequence and corresponding amino acid sequence of the 34 kd HGF variant, HGF/NK2. SEQ$_1$ ID NO:2 shows the amino acid sequence of the 34 kd HGF variant, HGF/NK2 only.

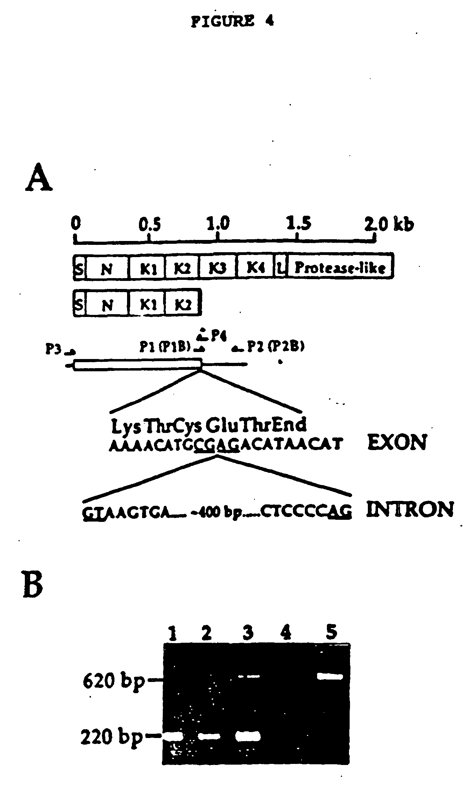

Figure 4 provides further characterization of a HGF/NK2 cDNA. (A) Schematic representation of the domain structures of HGF and HG F/NK2 (open boxes). The 1.2 kb cDNA clone pH45, comprised of a coding (open bar) and untranslated regions (solid lines). Arrows represent the positions and directions of PCR primers utilized. The cDNA and the predicted amino acid sequences of HGF/NK2 (EXON) at the point of divergence with HGF are shown with the splice site indicated. The corresponding genomic region (INTRON) includes a ~400bp intron with the consensus splicing signals at the exon-intron boundaries underlined. Abbreviations are: S, signal peptide; N, N-terminal domain; K1-K4, kringle 1 to 4; and L, linker region. Primers are:

| | |
|---|---|
| **P1** | **agtactgtgcaattaaaacatgcg** |
| **P2** | **gtagaaaaatgattgtatggactgcta** |
| **P1(B)** | **atggatccagtactgtgcaattaaaacatgcg** |
| **P2(B)** | **atggatccgtagaaaaatgattgtatggactgcta** |
| **P3** | **aggcactgactccgaacaggattctttcacccaggcatct cctcc** |
| **P4** | **atggatccttatgtctcgcatgtttaatgcaca** |

(B) Detection of HGF/NK2 transcript by PCR amplification. Samples included positive control plasmid pH45 (lane 1), RNAs from M426 (lane 2), SK-LMS-1 (lane 3), and B5/589 (lane 4); and genomic DNA from M426 cells (lane 5). Primers P1 and P2 were used in the amplification reactions and PCR fragments (220 and 620 bp) generated are indicated. The faint 620 bp band in lane 3 is indicative of unprocessed HGF RNA or genomic DNA in the SK-LMS-1 RNA preparation.

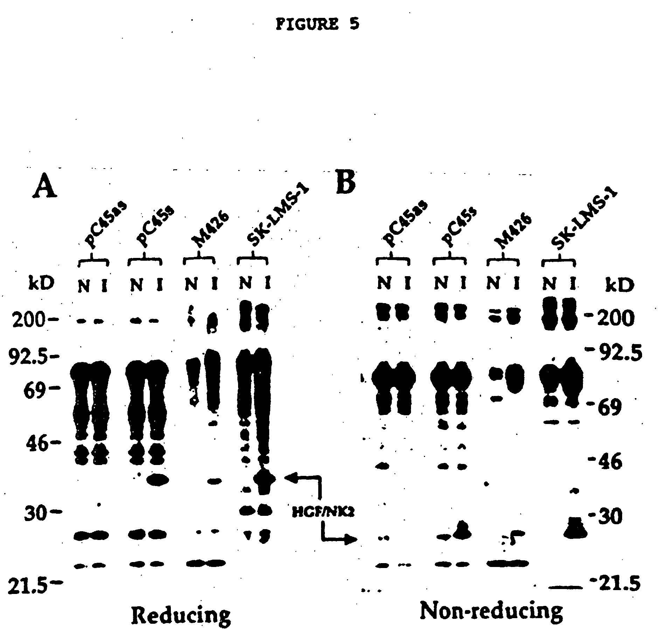

Figure 5 demonstrates the expression of HGF/MK2 cDNA in COS-1 cells. Conditioned medium from COS-1 cells transfected with plasmid pC45as (antisense construct) or pC45s (sense contruct) as well as M426 and SK-LMS-1 cells were immunoprecipitated with nonimmune (N) or HGF antiserum (I). Samples were analyzed under both reducing (A) and nonreducing (B) conditions. Specific HGF/NK2 immunoreactive species are indicated by arrows.

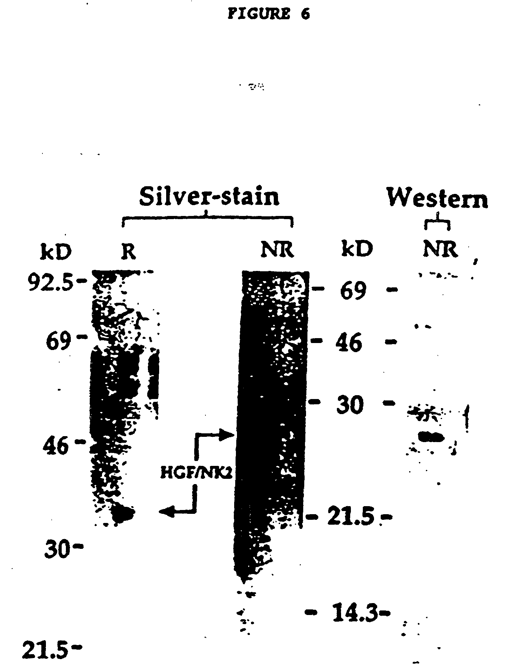

Figure 6 shows purified naturally occurring HGF/NK2. HGF/NK2 was purified from conditioned medium of SK-LMS-1 cells as described in the Examples. Aliquots from selected fractions eluted from a TSK sizing column were analyzed on 10% SDS-PAGE under reducing condtions (R) or 14% SDS-PAGE under non-reducing conditions (NR) and detected by the silver-stain technique. HGF/NK2 was visualized as a single band migrating at 34 and 28 kD, respectively (Arrows). Higher molecular weight artifactual bands were observed under reducing conditions. An identical sample was subjected to 14% SDS-PAGE under non-reducing conditions and immunoblotted with HGF antiserum.

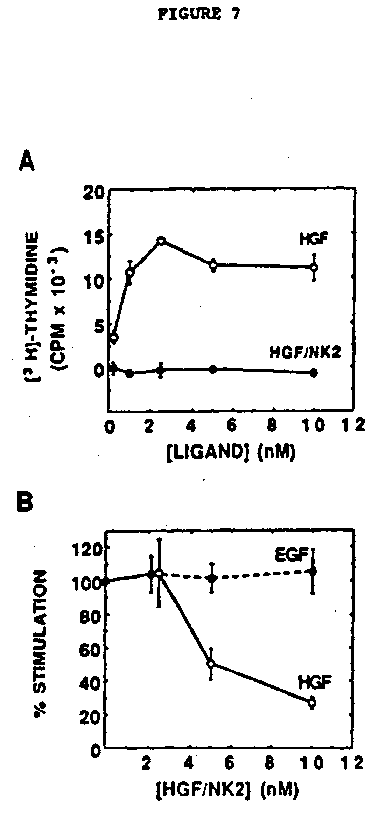

Figure 7 depicts the analysis of HGF/NK2, biological activity. (A) Comparison of DNA synthethis stimulated by HGF (-O-) and HGF/NK2 (-●-). B5/589 cells were exposed to increasing concentrations of either protein and [3H]-thymidine incorporation was measured as described in the experimental procedures.
(B) Effect of HGF/NK2 on HGF (-O-) and EGF (--●--) - induced [3H]-thymidine incorporation by B5/589 cells. Results are expressed as percentage of stimulation in the absence of HGF/NK2. HGF- and EGF-treated cells were tested at growth factor concentrations (0.2nM and 0.3nM, respectively) in the linear range of their dose-response curves.
Typically, the stimulation was 10,000-20,000 cpm with a background of 2000 cpm.
For both (A) and (B), each data point is the mean ± standard deviation of triplicate measurements; when no error bar is shown, the error was less than the symbol size.

Figure 8 shows the cross-linking and competition analysis of HGF/NK2 to the HGF receptor. [125I]-HGF/NK2 was incubated with B5/589 cells in the presence or absence of HGF /NK2, HGF, or EGF at the concentrations indicated for 45 minutes at 22°C.
Cultures were then washed with HEPES saline and incubated for 15 minutes with the cross-linking agent, disuccinimidyl suberate. Total cell lysates were resolved by 6.5% SDS-PAGE under reducing conditions and the dried gel was exposed to X-ray film at -70°C for 32 days.

Figure 9, SEQ ID NO: 3 and SEQ ID NO:4 show the cDNA coding sequence and corresponding amino acid sequence of the HGF variant encoded by the 1.5 and 2.2 kb transcripts, HGF/NK1. SEQ ID NO:4 shows the amino acid sequence of the HGF variant encoded by the 1.5 and 2.2 kb transcripts, HGF/NK1.

Detailed Description of the Invention

The present invention relates to a truncated form of hepatocyte growth factor (HGF), encoded by alternative HGF transcripts which specify a sequence that includes the N-terminal and first two kringle domains. This protein specifically antagonizes the mitogenic activity of HGF. The present invention also relates to another truncated form of HGF encoded by alternative HGF trancripts which specify the sequence that includes the N-terminal and only the first kringle domain (HGF/NK1). The invention further relates to diagnostic and therapeutic applications of the small HGF inhibitor.

A principle embodiment of the present invention relates to a truncated variant of HGF that is synthesized in cells that also normally synthesize HGF. One such HGF variant is characterized by a molecular weight of about 34 kd as determined by SDS-PAGE under reducing conditions. The molecule lacks mitogenic activity but specifically inhibits HGF induced mitogenesis by competing with the growth factor for binding to the HGF receptor.

The HGF variant and HGF protein sequences are > 99% identical throughout the entire length of the smaller HGF variant molecule. The truncated HGF and allelic variations thereof represent the product of an alternative transcript derived either from the same genetic locus encoding HGF or from a recently duplicated gene copy. This conclusion is supported by findings that not only the NK2 coding sequence but its upstream 5'-untranslated region are identical to that of the HGF cDNA. Further evidence shows that the k2 (kringle two) sequence is contiguous in human genomic DNA with the exon containing the termination codon and polyadenylation signal for the NK2 transcript (Figure 4 (A)).

The HGF variant protein to which the invention relates can be isolated from conditioned medium of a human Leiomyosarcoma cell line as well as other cell lines, for example, M426 fibroblast line, substantially free from other proteins.

Following the instructions presented herein, an active form of inhibitory HGF variant of the present invention can be obtained by a combination of protein purification steps that include concentrating the conditioned medium, applying the concentrate to heparin supports, for example, heparin-Sepharose resins, and eluting the HGF variant with an increasing salt gradient. Purified HGF variant is realized after the heparin bound eluate is fractionated over a sizing column, for example, TSK-G3000, in order for the HGF variant to be separated from any remaining components in the eluate. Alternatively, the variant can be produced chemically or recombinantly using methods known in the art.

The present invention also relates to the cDNA clones that encode the truncated HGF variants, HGF/NK2 and HGF/NK1. By screening a M426 human lung fibroblast cDNA library with DNA probes specific for either the heavy or light chain region of HGF, four cDNA clones were identified that hybridized to the heavy but not the light chain probe. Two of these four clones, having inserts of 1.2 or 1.6 kb, contain the coding sequence for the inhibitory HGF variant, HGP/NK2; they differed from each other in length of their 3' -untranslated sequence. However, the other two clones contained inserts of 1.5 and 2.2 kb, respectively, and each of which encoded only the N-terninal and first kringle domain; they differed from each other in their 3'-untranslated region. The resultant truncated form of HGF, HGF/NK1, has a predicted molecular weight of approximately 20 kilodaltons and is anticipated to have specific HGF inhibitory activity like HGP/NK2. The Northern blot analysis of HGF expression in M426 and SK-LMS-1 human cell lines revealed a weak 2.2 kb band as well as a diffuse signed at 1.3-1.5 kb (Figure 2) which probably represent the transcripts corresponding to these low abundance cDNAs.

The present invention further relates to recombinant DNA molecules comprising a vector and DNA fragment which encodes either of the human truncated HGF variants, HGF/NK1 or HGF/NK2.

Possible vectors include plasmids, for example, pCDV-1 and other vectors such as pZIPneo, known in the art that either transiently (pCDV-1) or stably (pZIPneo) transform host cells in a manner which allows expression of the HGF variant. Examples of appropriate eukaryotic host cells include, for example, mouse fibroblasts and monkey epithelial cells. The baculovirus as well as other eukaryotic or prokaryotic expression systems could be adapted for the production of the HGF variant.

The present invention also relates to therapeutic applications of truncated forms of HGF to which the invention relates, such as HGF/NK2, which has been shown to inhibit the mitogenic activity of HGF. Use of a specific inhibitor of HGF action can be beneficial in treating proliferative disorders, including both cancer and non-malignant conditions like benign prostatic hypertrophy, when HGF stimulation is excessive. The inhibitory HGF variant of the invention can be administered by different routes, for example, topical, oral or intravenous, to patients with such proliferative disorders. It is expected that providing therapeutic amounts of inhibitory HGF variant will return cell proliferation to normal levels. Alternatively, situations in which the production of the inhibitory HGF variant(s) is inappropriately high with a resultant impairment in cell proliferation or renewal can be addressed by specifically blocking the synthesis or action of these molecules (i.e., by use of antisense oligonucleotides to the unique 3'-untranslated sequences or antibodies specific for the inhibitory molecules).

The present invention also relates to methods of diagnosing pathological conditions in which cell growth is either impaired or excessive, due at least in part to the level of expression of HGF and its inhibitory variant(s). Fluctuating levels of these transcripts, particularly of the 1.3 kb transcript relative to the transcript encoding mitogenically active HGF, have been observed in different cell lines in a manner which may correlate with a functional role in regulating proliferation. For instance, the 1.3 kb transcript is expressed at relatively low levels in an embryonic fibroblast line which supports active cell division, but the transcript is present at much higher levels in an adult fibroblast strain which is likely to provide a more attenuated stimulus of cell renewal. As one skilled in the art will appreciate, increased protein production can result from increased levels of corresponding mRNA transcripts. Using DNA fragments derived from the cDNAs of HGF variants and standard methodology known in the art, HGF variant transcripts can be detected as shown in Figure 2. Detection may be performed with extracted RNA or by in situ hybridization using the DNA fragments or RNA fragments derived therefrom.

In another detection method for diagnosing pathological conditions in which cell growth is either impaired or excessive, a biological sample from a patient is contacted with antibodies specific for HGF and/or specific HGF variants. Using standard methodologies well known in the art, the antibody-protein complex can be detected, for example, by immunoprecipitation and SDS-polyacrylamide gel electrophoresis (Figure 1), immunoblotting (Figure 6), enzyme-linked immunosorbent assay (ELISA) or immunohistochemistry.

Certain aspects of the invention are described in greater detail in the non-limiting Examples that follow.

Examples

The protocols described below are referenced in the following Examples.

Cell culture

Cells including the M426 human embryonic lung fibroblast (S.A. Aaronson and G.J. Todaro *Virology* **36**: 254-261 (1968), SK-LMS-1 human leiomyosarcoma (J. Fogh and G. Trempe *In: Human Tumor Cells In vitro,* J. Fogh (ed.), Plenum Press, New York 115-159), and COS-1 monkey kidney epithelial (Gluzman et al. *Cell* **23**:175-182 (1981) cell lines were maintained in Dulbecco's modified Eagle's medium (DMEM) suplemented with 10% fetal calf serum (Bethesda Research Laboratories). B5/589 human mammary epithelial cells (M.R. Stampfer and J.C. Bartley *Proc. Natl. Acad. Sci. U.S.A* **82**:2394-2398 (1985) were grown as described (Rubin et al., *Proc. Natl. Acad.Sci.USA* **86**:802 (1989))

Mitogenic assays

DNA synthesis was measured as previously described (Rubin et al., *Proc. Natl. Acad. Sci. USA* 86:802 (1989)). Ninety-six well microtiter plates were precoated with human fibronectin at 1 $\mu$g/cm$^2$ prior to seeding with B5/589 cells. [$^3$H]-thymidine incorporation was determined during a 6-hr period beginning 16 hr after addition of samples. Trichloroacetic acid-insoluble DNA was collected and counted. HGF used in this study was purified in this laboratory as has been reported (Rubin et al., *Proc. Natl. Acad. Sci. USA* **88**:415 (1991)), and human recombinant EGF was purchased from Upstate Biotechnology Inc.

Immunoprecipitation

Cells in 100mm tissue culture plates were labeled with 0.1mCi/ml of [$^{35}$S]-methionine and cysteine (spec. act. 1150Ci/ml; Du Pont-New England Nuclear) in 50$\mu$g/ml of heparin for 4 hrs as previously described (Rubin et al., *Proc. Natl. Acad. Sci. US4* **88**:415 (1991)). Conditioned medium was concentrated 20-fold in Centricon-10 microconcentrator (Amicon) and immunoprecipitated with nonimmune or HGF neutralizing antiserum. Immunoprecipitates were absorbed onto Gamma-bind G agarose (Genex) and washed three times with 10mM Tris-HCl buffer containing 150mM NaCl, 0.05% Tween-20, 0.1% SDS, 1% MP-40, 1mM EDTA, and 10mM KC1. Samples were analyzed under reducing (with 100mM B-mercaptoethanol) and non-reducing conditions on 10% or 14% SDS-PAGE. Gels were fixed, treated with enlightening solution (New England Nuclear), dried, and exposed to Kodak AR film at -70°C.

Northern analysis

Poly(A)$^+$RNA was isolated by oligo-dT columns as described (Maniatis et al. *Molecular cloning. A Laboratory Manual* Cold Spring Harbor, New York: Cold Spring Harbor Laboratory (1982)). Following electrophoresis in 1% denaturing formaldehyde agarose gels, samples were transferred onto nitrocellulose filters (Maniatis et al. *Molecular cloning. A laboratory Manual* (Cold Spring Harbor, New York: Cold Spring Harbor Laboratory (1982)). Blots were hy-

bridized to [$^{32}$P]-labeled randomly-primed DNA probes in 40% formamide, 6x SSC, 5x Denhardt's solution, 50mM sodium phosphate (pH6.8), and 250μg/ml of sonicated salmon sperm DNA at 42°C for 12 hrs. After hybridization, filters were washed twice in ix SSC, 0.1% SDS at room temperature. The final wash was carried out in 0.1×SSC, 0.1% SDS at 55°C. Filters were dried and exposed to X-ray films for 5-8 days at -70°C. Hybridization probes were generated by PCR and purified on low-melting temperature agarose gels. The nucleotide sequence of each probe was numbered according to the HGF sequence of Miyazawa et al. *Biochem. Biophys. Res. Commun.* **163**:967-973 (1989) as follows:

| H/L | (heavy and light chains) | -24 to +2187 |
| H | (heavy chain) | +189 to +1143 |
| L | (light chain) | +1475 to 2122 |

### cDNA cloning and sequencing

Approximately 1x10$^6$ phage plaques from an M426 cDNA library (Finch et al. *Science* **245**:752-755 (1989) were plated, and duplicate filters were hybridized separately to radiolabeled probes H and L (see above) under conditions identical to those described for Northern analysis. Restriction mapping of plaque purified positive clones was performed using standard procedures (Maniatis et al. *Molecular cloning. A laboratory Manual* (Cold Spring Harbor, New York: Cold Spring Harbor Laboratory (1982)). cDNA inserts were excised and subcloned into the M13mp18 vector for sequencing analysis by the dideoxy chain-termination method (Sanger et al., *J. Mol. Biol.* **143**: 161-178. (1977).

### PCR analysis

For PCR of mRNA, 1μg of poly(A)$^•$ RNA was first reverse-transcribed by avian myeloblastosis virus(AMV) reverse transcriptase (Bethesda Research Laboratories) using random hexamers (Pharmacia) as primers (Noonan et al. *Nucleic Acids Res.* **16**:10366 (1988)). Eight percent (-80ng) of the first-strand cDNA products were used directly in PCR (Saiki et al. *Science* **230**:1350-1354 (1985)). For routine PCR, 80ng of cDNA, 0.5μg of cellular DNA, and 10ng of plasmid DNA were subjected to 30 cycles of amplification using primers P1 and P2 (see Figure 4). Cycling conditions were: 1 minute at 94°C, 2 minutes at 60°C, and 3 minutes at 72°C. Aliquots (10%) of each reaction mixture were anlyzed on 3% agarose gel. For PCR cloning of genomic DNA, PCR was carried out with BamHI linker-primers P1B and P2B (Figure 4) and amplified DNA fragments were digested with BamHI. The resultant BamHI fragments were purified on low-melting temperature agarose gel and subcloned into the M13mp18 vector for sequencing analysis.

### Transient expression in COS-1 cells

The NK2 coding sequence was generated by PCR using BamHI linker-primers, P3 and P4 (Figure 4) and subcloned into the BamHI site of the vector pCDV-1 (Okayama et al. *Mol. Cell. Biol.* **3**: 280-289 (1983)) in both orientations. The NK2 insert in a selected construct was sequenced to ensure that the PCR product was correct. Ten μg of each plasmid DNA was transfected by the calcium phosphate precipitation method (Wigler et al. *Cell* **11**:223-232 (1977)) into COS-1 cells (Y. Gluzman *Cell* **23**:175-182 (1981)). At 48 hrs, proteins in conditioned medium were processed for labeling, immunoprecipitation and 10% SDS-PAGE under reducing and non-reducing conditions as described above.

### Protein purification

Six liters of conditioned medium from SK-LMS-1 cells grown in 175-cm$^2$ T flasks were prefiltered through a 0.5-μm filter (Millipore HAWP 142 50), and concentrated to 300ml by a Pellicon cassette system having a 10 kD molecular mass cutoff (Millipore PTGC 000 05). Concentrated medium was loaded onto heparin-Sepharose resin (4 ml. bed volume, LKB/Pharmacia) that had been equilibrated in 20mM Tris-HCl, pH7.5/0.3 M NaCl. The sample was eluted with a modified linear gradient of increasing NaCl concentration. Aliquots from each fraction were subjected to immunoblot analysis with antiserum raised against HGF (final dilution 1:500) to identify the presence of HGF/NK2. Pooled fractions were further resolved on a TSK G3000 sizing column (LKB/Pharmacia) in 20mM Tris-HCl, pH6.8/1.0 M NaCl. The purity and identity of the HGF/NK2 protein were determined by silver-stain analysis (Merril et al. *Science* **211**: 1437-1438 (1981)) and immunoblotting under reducing and non-reducing conditions. Fractions containing >95% of HGF/MK2 were selected for biological analysis. Protein concentration was estimated by optical density, assuming

$$A^{1\%}_{214} = 140.$$

Affinity cross-linking

TSK-purified HGF/NK2 was iodinated by the chloramine-T method (W.M. Hunter and F.C. Greenwood *Nature* **194**:495-496 (1962)) and represented over 99% of the labeled material in the preparation as determined by SDS-PAGE analysis. Affinity cross-linking experiments were performed on 6-well plates seeded with B5/589 cells at a density of $5 \times 10^5$ per well. To each well, HGF/NK2 ($5 \times 10^5$ cpm at a specific activity of $\sim 200$ µCi/µg was added with or without cold competitors in HEPES binding buffer (100mM HEPES, 150mM NaCl, 5mM KC1, 1.2mM $MgSO_4$, 8.8mM dextrose, 2µg/ml heparin, and 0.1% BSA, pH7.4). Following incubation at room temperature for 45 minutes, cells were washed twice in cold HEPES sa line (pH 7.4). Disuccinimidyl suberate (Pierce) in dimethyl sulfoxide was added to a final concentration of 250 µM and incubated for 15 min. Samples were then quenched with 100 µl of 20mM Tris /100mM glycine /1mM EDTA for 1 minute and rinsed in HEPES saline. Cells were extracted with Laemmli sample buffer and resolved on 6.5% SDS-PAGE under reducing conditions.

Example 1.     Detection of a small naturally occurring HGF immunoreactive species and its putative transcrunpt

Previous studies demonstrated that HGF is synthesized as a single-chain polypeptide with an apparent molecular mass (Mr) of 87,000 (87 kD) . It can be cleaved into a heterodimeric form consisting of a heavy- (M, 60 kD) and light-chain (M, ~30 kD) held together by disulfide bonds. Neutralizing antiserum against purified HGF was used to immunoprecipitate proteins in conditioned medium from metabollically labeled M426 human embyonic lung fibroblasts. When sodium dodecyl sulfate polyacrylamide gel electrophoresis (SDS-PAGE) was performed under reducing conditions, the single-chain form (HGFp87) was the predominant species. While there was no evidence of the processed heavy-and light- chains, low levels of a HGF immunoreactive molecule, of M, -34 kD (p34) were observed (Figure 1). Pulse chase experiments showed that both HGFp87 and p34 shared similar kinetics of synthesis and secretion arguing against the likelihood that p34 was a HGFp87 degradation product. When the same experiment was performed with another HGF-producer, a leiomyosarcoma cell line (SK-LMS-1), a similar pattern was seen except that p34 was relatively more abundant (Figure 1).
To gain further understanding of the relationship between HGFp87 and p34, poly (A)• RNA was prepared from M426 and SK-LMS-1 cells and subjected to Northern blot analysis using the full-length HGF coding sequence as probe. As shown in Figure 2, two major transcripts of 6.0 and 3.0 kilobases (kb) were detected in both lines. Each of these transcrupts has previously been shown to encode the full-length growth factor (Rubin et al., *Proc. Natl. Acad. Sci. USA* **88**: 415 (1991)). A third HGF. hybridizing RNA of ~ 1.3 kb was present at a relatively low level in M426 cells, but was expressed at higher levels in SK-LMS-1 cells. This pattern was consistant with the relative levels of p34 observed in the two cell lines, suggesting that p34 might be encoded by the novel 1.3 kb transcript. Based on the fact that the complete HGF coding sequence is ~ 2.0 kb, the 1.3 kb transcript could only represent a portion of this region. To test this, the same Nothern blot was hybridyzed separately with probes derived from either the N-terminal heavy-chain or the C-terminal light-chain. Whereas both probes were able to detect the 6.0 and 3.0 kb transcripts, only the heavy-chain probe was capable of recognizing the 1.3 kb message (Figure 2). These results suggested that this RNA species encoded a truncated version of the HGF molecule containing sequences from its N-terminal region.
Other faint bands were also detected in the Northern blots hybridized with probes derived from HGF (Figure 2), including one at approximately 2.2 kb. The significance of this observation became apparent after further study (see Example 2).

Example 2.     Isolation of HGF cDNA clones encoding only the N-terminal and first one or two kringle domains

In an attempt to isolate cDNA clones corresponding to the 1.3 kb transcript, an M426 cDNA library was differentially screened with both HGF heavy- and light-chain probes. Clones that specifically hybridized to the heavy- but not the light-chain probe were plaque purified. Based, on the sizes and physical maps of the inserts, one cDNA clone, pH45 with an insert of ~1.2 kb was selected for sequencing. As shown schematically in Figure 4A, clone pH45 depicted a transcript of 1199 basepairs (bp) composed of a short 5'-untranslated region of 75 bp, an open reading frame of 870 bp and a 254 bp 3'-untranslated region containing a polyadenylation signal, AATAAA. The open reading frame predicted a 290 amino acid truncated version of HGF consisting of a signal peptide, an N-terminal domain (N), and the first two kringle domains (K1 and K2) with a calculated Mr of -30kD excluding the signal peptide. This sequence, which is designated NK2 was identical to that of HGF cDNA until it diverged at a point which coincided precisely with the end of the K2 domain. The NK2 open reading frame continued for two additional amino acids followed by an in-frame stop codon (TAA) (Figure 3, SEQ ID NO:1 and 4A).
To ascertain the authenticity of the cDNA clone, polymerase chain reaction (PCR) analysis was performed with primers P1 and P2 (Figure 4A), the latter of which was specific for the NK2 transcript. Figure 4B shows the existence of the predicted 220 bp PCR fragment in RNA of M426 and SK-LMS-1 cells but not in B5/589 cells, which lack detectable HGF transcripts. The gene structure of this region was further analyzed by amplifying the corresponding genomic sequence using the same PCR primers (Figure 4B). Sequencing of the PCR product revealed a ~400 bp intron with the consensus splice donor/acceptor sequences CG/GT and AG/AG at the intron-exon boundaries, which aligned precisely with the predicted splice junction in the NK2 cDNA clone (Figure 4A). Thus, the 1.3 kb NK2 transcript is likely generated during precursor RNA processing by joining of the K2 exon to an alternative exon containing a termination codon instead of the K3 exon.

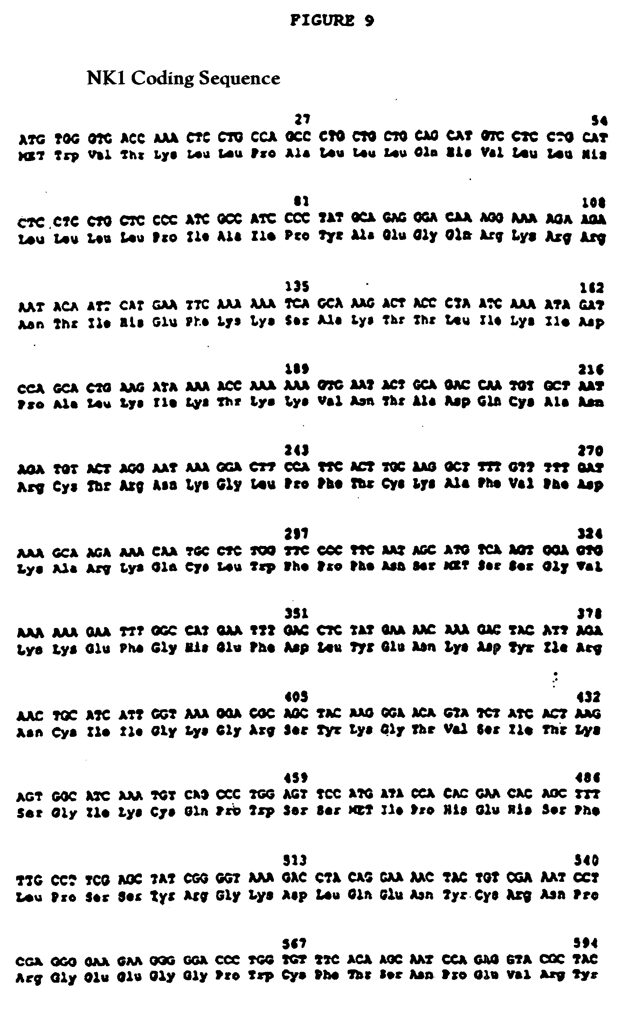

Using the differential screening strategy described above, three additional cDNA clones that specifically hybridized to the HGF heavy as opposed to the light chain probe were isolated from the M426 library. One of these was ~1.6 kb and contained the coding sequence for HGF/NK2; it differed from the 1.3 kb transcript only insofar as it included a longer stretch of 3' untranslated sequence. However, the other two inserts, one 1.5 and the other 2.2 kb, encoded only the N-terminal and first kringle domain; they differed from each other in their 3' untranslated regions. The coding sequence of one of these NK1 cDNAs is presented in Figure 9 and SEQ ID NO: 3. As noted in the previous section, a close examination of the HGF hybridization pattern in Northern blot analysis revealed a weak 2.2 kb band as well as a diffuse signal at 1.3 - 1.6 kb (Figure 2) which probably represents the transcripts corresponding to these low abundance cDNAs.

Example 3.      Recombinant expression of HGF/NK2 cDNA identifies its product as the small HGF cross-reactive species

In order to test whether the NK2 transcript encodes the p34 protein detected in M426 and SK-LMS-1 cells, the NK2 coding region was subcloned into the expression vector, pCDV-1, in both anti-sense (pC45as) and sense (pC45s) orientations. Conditioned medium of COS-1 cells transfected with either construct was collected and immunoprecipitated with HGF neutralizing antibodies followed by SDS-PAGE analysis. As shown in Figure 5A, pC45s transfected COS-1 cells secreted a 34 kD HGF immunoreactive recombinant protein (rHGF/NK2) not detected when COS-1 cells were transfected with the pC45as construct. The size of this protein corresponded closely to that of p34 from M426 and SK-LMS-1 cells (Figure 5A). When the same experiment was performed under non-reducing conditions, the mobility of both recombinant and naturally occuring p34 shifted to an apparent Mr of - 28 kD (Figure 58), providing further evidence that p34 and rNK2 were structurally indistinguishable.

The next experiment compared the heparin-binding properties of p34 and rHGF/MK2. Conditioned medium collected from SK-LMS-1 and pC45s-transfected COS-1 cells were each applied to heparin-Sepharose resin, and bound proteins were eluted with increasing NaCl concention. When individual fractions were analyzed by immunoblotting with anti-HGF serum, both p34 and rHGF/NK2 shared the same chromatographic profile with an elution peak at ~ 1.0M NaCl. Taken together, these findings indicated that the p34 protein secreted by M426 and SK-LMS-1 cells represented a truncated version of HGF expressed from the NK2 transcript. Thus, the p34 protein was designated as HGF/NK2.

The NK2 coding region was also subcloned into the pZ1Pneo expression vector and subsequently transfected into NIH/3T3 mouse fibroblasts. The metabolically labeled protein was detected in the condition medium of transfected cells, but levels were not sufficient for preparative work.

Example 4.      Purified HGF/NK2 is a specific inhibitor of HGF mitogenic activity

To investigate its biological activity, HGF/NK2 was purified from culture f luids of SK-LMS-1 cells by a three-step procedure combining ultrafiltration, heparin-Sepharose and TSK sieving chromatography. The purified protein exhibited the characteristic mobility shift under non-reducing and reducing conditions and was immunoreactive with anti-HGF serum, thereby confirming its identity as HGF/NK2 (Figure 6).

To test the mitogenic activity of HGF/NK2, a human mammary epithelial cell line, B5/589 was used as the target cell. While HGF stimulated [$^3$H]-thymidine incorporation with a half-maximal effect at ~0.25nM, under identical conditions HGF/NK2 at concentrations as high as 10nM caused no enhancement of DNA synthesis (Figure 7A). In view of their structural similarity, the possibility that HGF/NK2 might act as a specific inhibitor of HGF was also tested. When DNA synthesis induced by HGF was measured in the presence of increasing HGF/NK2 concentrations, a dose-dependent inhibition of [$^3$H]-thymidine incorporation was observed (Figure 7B). To achieve a 50% inhibition, a 10- to 20-fold molar excess of HGF/NK2 over HGF was required. Similar results were obtained when human melanocytes were used as target cells. Moreover, the inhibition was specific for HGF since HGF/MK2 did not impair the mitogentic activity of epidermal growth factor (EGF)(Figure 7B).

Example 5.      Competitive binding of HGF/NK2 and HGF to the HGF receptor

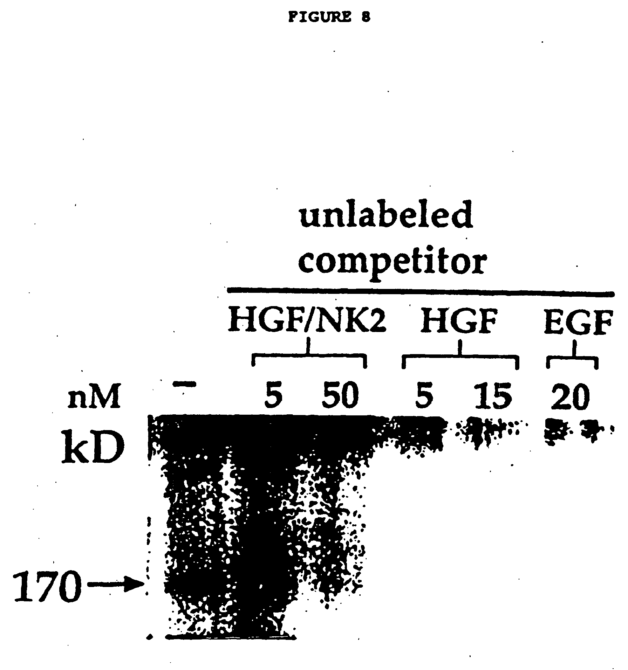

It was recently demonstrated that the *c-met* protooncogene product, a membrane-spanning tyrosine kinase, is the cell surface receptor for HGF (Bottaro et al., *Science* **251**: 802 (1991)). To elucidate the mechanism by which HGF/NK2 acted as an antagonist of HGF mitogenic activity, cross-linking studies of [$^{125}$I]-HGF/NK2 to B5/589 cells were performed. As shown in Figure 8, a single major cross-linked species of 170 kD was detected under reducing conditions. This band corresponds to the 145 kD β-subunit of the processed *c-met* product cross-linked to HGF/NK2 (Bottaro et al., *Science* **251**:802 (1991)). Increasing concentrations of either unlabeled HGF/NK2 or HGF effectively competed with the labeled ligand in the cross-linking reaction. On a molar basis, HGF was estimated to be 3 to 5 times more effective than HGF/NK2 itself as a competitor of [$^{125}$I]-HGF/NK2 cross-linking. Under the same conditions, EGF failed to block HGF/NK2 cross-linking (Figure 8). All of these findings demonstrate specific competitive binding of HGF/NK2 and HGF to the same cell surface receptor molecule.

## Claims

1. A hepatocyte growth factor (HGF), variant protein in truncated: form which is consisting of the N-terminal, and either the first kringle domain or the first two kringle domains only of HGF, and which specifically inhibits HGF-induced mitogenesis by binding to the HGF receptor.

2. The HGF variant protein of claim 1 which has both of the first two kringle domains.

3. The HGF variant protein of claim 1 which has only the first of the first two kringle domains.

4. The HGF variant protein of claim 1 or 2 which has an approximate molecular weight of 34kD

5. The HGF variant protein of claim 1 or 3 which has an approximate molecular weight of 20kD.

6. A recombinantly produced protein comprising an amino acid sequence of a HGF variant protein, which is consisting of the N-terminal and only the first kringle domain of HGF (HGF/NK1) or only the first two kringle domains of HGF (HGF/NK2).

**7.** A recombinantly produced protein of claim 6 having an amino acid sequence given in Figure 3.

**8.** The protein of claim 6, wherein the HGF/NK1 comprises the amino acid sequence of figure 9.

**9.** A DNA segment encoding said HGF variant protein according to one of claims 1 to 8.

**10.** The DNA segment of claim 9, encoding a HGF variant protein, which is consisting of the N-terminal and only the first kringle domain of HGF (HGF/NK1).

**11.** The DNA segment of claim 9 or 10, wherein the DNA segment encodes a HGF protein variant having an approximate molecular weight of 20kD.

**12.** The DNA segment of claim 9, wherein the DNA segment is encoding a HGF variant protein which is consisting of the N-terminal and only the first two kringle domains of HGF (HGF/NK2).

**13.** The DNA segment of claim 9 or 12, wherein the DNA segment encodes an HGF protein variant having an approximate molecular weight of 34kD

**14.** The DNA segment according to claim 9 wherein said variant protein has the amino acid sequence defined in Figure 3.

**15.** A recombinant DNA molecule comprising a DNA segment according to one of claims 9 to 14 and a vector.

**16.** A host cell stably or transiently transfected or transduced with the recombinant DNA molecule according to claim 15 in a manner allowing expression of said protein encoded by said DNA fragment.

**17.** A method of producing a recombinant 34 kD HGF variant protein comprising culturing host cells according to claim 16 in a manner allowing expression of said protein and isolating said protein from said host cells.

**18.** A method of producing a recombinant 20 kD HGF variant protein comprising culturing host cells according to claim 16 in a manner allowing expression of said protein and isolating said protein from said host cells.

**19.** A method of producing a HGF variant protein as defined according to one of claims 1 to 8 from cultured cells comprising the following steps :

(i) culturing HGF variant protein-producing cells in culture medium under conditions such that HGF variant protein is produced ;
(ii) concentrating said culture medium so that a concentrate is formed ;
(iii) contacting said concentrate with heparin under conditions such that HGF variant protein in said concentrate binds to the heparin whereby a heparin-HGF variant protein complex is formed ;
(iv) separating said heparin-HGF variant protein complex from said concentrate;
(v) treating said heparin-HGF variant protein complex under conditions such that said HGF variant protein dissociates from the heparin so that a solution of free HGF variant protein is formed; and
(vi) fractionating said solution by sizing chromatography so that HGF variant protein is separated from the remaining components.

**20.** A method of producing HGF variant protein from cultured cells according to claim 16 wherein said HGF variant protein-producing cells are human leiomyosarcoma SK-LMS- 1 cells.

**21.** Use of the HGF variant protein according to one of claims 1 to 8 for the preparation of a composition for inhibiting the growth of cells, said composition containing a therapeutic amount of the HGF variant protein under conditions such that cell growth is inhibited.

**22.** A method of diagnosing growth disorders comprising the steps of:

(i) isolating mRNA from a biological sample,
(ii) contacting said mRNA with a DNA segment according to one of claims 9 to 14, and
(iii) detecting the presence of RNA-DNA hybrids.

23. A method of diagnosing growth disorders by in situ hybridization comprising the steps of :

    (i) contacting mRNA contained in an isolated sample with a DNA segment according to one of claims 9 to 14 or a RNA fragment derived therefrom, and
    (ii) detecting the presence of the hybrids.

24. An in vitro method of inhibiting the growth of cells comprising contacting said cells in vitro with a therapeutic amount of the HGF variant protein according to anyone of claims 1 to 8 under conditions such that cell growth is inhibited.

**Patentansprüche**

1. Hepatozyten-Wachstumsfaktor (HGF) - Proteinvariante in verkürzter Form, die nur aus dem N-Terminus und entweder der ersten Kringeldomäne oder den ersten zwei Kringeldomänen von HGF besteht, und die die HGFinduzierte Mitogenese durch das Binden an den HGF-Rezeptor spezifisch inhibiert.

2. HGF-Proteinvariante gemäss Anspruch 1, die beide der ersten zwei Kringeldomänen hat.

3. HGF-Proteinvariante gemäss Anspruch 1, die nur die erste der ersten beiden Kringeldomänen hat.

4. HGF-Proteinvariante gemäss Anspruch 1 oder 2, die ein ungefähres Molekulargewicht von 34 kD besitzt.

5. HGF-Proteinvariante gemäss Anspruch 1 oder 3, die ein ungefähres Molekulargewicht von 20 kD besitzt.

6. Rekombinant hergestelltes Protein, umfassend eine Aminosäuresequenz einer HGF-Proteinvariante, die aus dem N-Terminus und nur der ersten Kringeldomäne von HGF (HGF/NK1) oder nur den ersten zwei Kringeldomänen von HGF (HGF/NK2) besteht.

7. Rekombinant hergestelltes Protein gemäss Anspruch 6 mit einer Aminosäuresequenz, die in der Fig. 3 angegeben ist.

8. Protein gemäss Anspruch 6, wobei HGF/NK1 die Aminosäuresequenz der Fig. 9 umfasst.

9. DNA-Segment, das die HGF-Proteinvariante gemäss einem der Ansprüche 1 bis 8 codiert.

10. DNA-Segment gemäss Anspruch 9, codierend die HGF-Proteinvariante, die nur aus dem N-Terminus und der ersten Kringeldomäne von HGF (HGF/NK1) besteht.

11. DNA-Segment gemäss Anspruch 9 oder 10, wobei das DNA-Segment eine HGF-Proteinvariante mit einem ungefähren Molekulargewicht von 20 kD codiert.

12. DNA-Segment gemäss Anspruch 9, wobei das DNA-Segment eine HGF-Proteinvariante codiert, die nur aus dem N-Terminus und den ersten beiden Kringeldomänen von HGF (HGF/NK2) besteht.

13. DNA-Segment gemäss Anspruch 9 oder 12, wobei das DNA-Segment eine HGF-Proteinvariante mit einem ungefähren Molekulargewicht von 34 kD codiert.

14. DNA-Segment gemäss Anspruch 9, wobei die Proteinvariante eine Aminosäuresequenz hat, die in der Fig. 3 dargestellt ist.

15. Rekombinantes DNA-Molekül, umfassend ein DNA-Segment gemäss einem der Ansprüche 9 bis 14 und einen Vektor.

16. Wirtszelle, die stabil oder transient mit dem rekombinanten DNA-Molekül gemäss Anspruch 15 transfiziert oder transduziert auf eine Weise ist, die die Expression des Proteins, das durch das DNA-Fragment codiert wird, erlaubt.

17. Verfahren zur Herstellung einer rekombinanten 34 kD HGF-Proteinvariante, umfassend das Kultivieren von Wirtszellen gemäss Anspruch 16 auf eine Weise, die die Expression dieses Proteins und das Isolieren dieses Proteins aus den Wirtszellen erlaubt.

18. Verfahren zur Herstellung einer rekombinanten 20 kD HGF-Proteinvariante, umfassend das Kultivieren von Wirtszellen gemäss Anspruch 16 in einer Weise, die die Expression dieses Proteins und das Isolieren dieses Proteins aus diesen Wirtszellen erlaubt.

19. Verfahren zur Herstellung einer HGF-Proteinvariante, wie in einem der Ansprüche 1 bis 8 definiert, aus kultivierten Zellen, umfassend die folgenden Schritte:

   (i) Kultivieren der HGF-Proteinvariante produzierenden Zellen in Kulturmedium unter Bedingungen, die so sind, dass die HGF-Proteinvariante hergestellt wird;

   (ii) Konzentrieren des Kulturmediums, so dass ein Konzentrat gebildet wird;

   (iii) Kontaktieren des Konzentrats mit Heparin unter Bedingungen, dass die HGF-Proteinvariante in diesem Konzentrat an Heparin bindet, wodurch ein Heparin-HGF-Proteinvarianten-Komplex gebildet wird;

   (iv) Abtrennen des Heparin-HGF-Proteinvarianten-Komplexes aus diesem Konzentrat;

   (v) Behandeln dieses Heparin-HGF-Proteinvarianten-Komplexes unter Bedingungen, dass die HGF-Proteinvariante von Heparin abdissoziiert, so dass eine Lösung aus freier HGF-Proteinvariante gebildet wird; und

   (vi) Fraktionieren dieser Lösung durch grössenabhängige Chromatografie, so dass die HGF-Proteinvariante von den übrigen Bestandteilen getrennt wird.

20. Verfahren zur Herstellung einer HGF-Proteinvariante aus kultivierten Zellen gemäss Anspruch 16, wobei die HGF-Proteinvarianten produzierenden Zellen humane SK-LMS-1-Leiomyosarkomazellen sind.

21. Verwendung der HGF-Proteinvariante gemäss einem der Ansprüche 1 bis 8 zur Herstellung einer Zusammensetzung zur Inhibierung des Wachstums von Zellen, wobei die Zusammensetzung eine therapeutische Menge der HGF-Proteinvariante enthält, unter Behandlungen, dass Zellwachstum inhibiert wird.

22. Verfahren zum Diagnostizieren von Wachstumsstörungen, umfassend die Schritte:

   (i) Isolieren von mRNA aus einer biologischen Probe,

   (ii) Kontaktieren der mRNA mit einem DNA-Segment gemäss einem der Ansprüche 9 bis 14, und

   (iii) Detektieren des Vorhandenseins von RNA-DNA-Hybriden.

23. Verfahren zum Diagnostizieren von Wachstumsstörungen durch in situ-Hybridisierung, umfassend die Schritte:

   (i) Kontaktieren von mRNA, die in einer isolierten Probe enthalten ist., mit einem DNA-Segment Gemäss einem der Ansprüche 9 bis 14, oder mit einem RNA Fragment davon erhalten.

   (ii) Detektieren des Vorhandenseins von RNA-DNA-Hybriden.

24. In vitro-Verfahren zur Inhibierung des Wachstums von Zellen, umfassend das Kontaktieren dieser Zellen in vitro mit einer therapeutischen Menge der HGF-Proteinvariante gemäss einem der Ansprüche 1 bis 8 unter Bedingungen, die das Zellwachstum inibieren.

**Revendications**

1. Protéine constituant un variant du facteur de croissance des hépatocytes (HGF) sous forme tronquée qui consiste en l'extrémité N-terminale et en le premier domaine kringle ou en les deux premiers domaines kringle seulement de HGF, et qui inhibe spécifiquement la mitogenèse induite par HGF en se liant au récepteur de HGF.

**2.** Protéine constituant un variant de HGF selon la revendication 1 qui a les deux premiers domaines kringle.

**3.** Protéine constituant un variant de HGF selon la revendication 1 qui a seulement le premier des deux premiers domaines kringle.

**4.** Protéine constituant un variant de HGF selon la revendication 1 ou 2 qui a une masse moléculaire approximative de 34 kD.

**5.** Protéine constituant un variant de HGF selon la revendication 1 ou 3 qui a une masse moléculaire approximative de 20 kD.

**6.** Protéine produite par recombinaison comprenant une séquence d'acides aminés d'une protéine constituant un variant de HGF, qui consiste en l'extrémité N-terminale et en seulement le premier domaine kringle de HGF (HGF/NK1) ou en seulement les deux premiers domaines kringle de HGF (HGF/NK2).

**7.** Protéine produite par recombinaison selon la revendication 6 ayant une séquence d'acides aminés donnée sur la figure 3.

**8.** Protéine selon la revendication 6 où le HGF/NK1 comprend la séquence d'acides aminés de la figure 9.

**9.** Segment d'ADN codant ladite protéine constituant un variant de HGF selon l'une quelconque des revendications 1 à 8.

**10.** Segment d'ADN selon la revendication 9 codant une protéine constituant un variant de HGF qui consiste en l'extrémité N-terminale et en seulement le premier domaine kringle de HGF (HGF/NK1).

**11.** Segment d'ADN selon la revendication 9 ou 10, où le segment d'ADN code un variant de protéine HGF ayant une masse moléculaire approximative de 20 KD.

**12.** Segment d'ADN selon la revendication 9, où le segment d'ADN code une protéine constituant un variant de HGF qui consiste en l'extrémité N-terminale et en seulement les deux premiers domaines kringle de HGF (HGF/NK2).

**13.** Segment d'ADN selon la revendication 9 ou 12 où le segment d'ADN code un variant de protéine HGF ayant une masse moléculaire approximative de 34 kD.

**14.** Segment d'ADN selon la revendication 9, où ladite protéine constituant un variant a la séquence d'acides aminés définie sur la figure 3.

**15.** Molécule d'ADN recombiné comprenant un segment d'ADN selon l'une des revendications 9 à 14 et un vecteur.

**16.** Cellule hôte transfectée ou transduite de manière stable ou transitoire avec la molécule d'ADN recombiné selon la revendication 15 d'une manière permettant l'expression de ladite protéine codée par ledit fragment d'ADN.

**17.** Procédé de production d'une protéine constituant un variant de HGF de 34 kD recombinée en prenant la culture de cellules hôtes selon la revendication 16 d'une manière permettant l'expression de ladite protéine et l'isolement de ladite protéine à partir desdites cellules hôtes.

**18.** Procédé de production d'une protéine constituant un variant de HGF de 20 kD recombinée comprenant la culture de cellules hôtes selon la revendication 16 d'une manière permettant l'expression de ladite protéine et l'isolement de ladite protéine à partir desdites cellules hôtes.

**19.** Procédé de production d'une protéine constituant un variant de HGF selon l'une quelconque des revendications 1 à 8 à partir de cellules cultivées comprenant les étapes suivantes :

(i) culture de cellules produisant une protéine constituant un variant de HGF dans un milieu de culture dans des conditions telles que la protéine constituant un variant de HGF est produite ;
(ii) concentration dudit milieu de culture de sorte qu'un concentré est formé ;
(iii) mise en contact dudit concentré avec de l'héparine dans des conditions telles que la protéine constituant un variant de HGF dans ledit concentré se lie à l'héparine si bien qu'il se forme un complexe héparine-protéine constituant un variant de HGF ;

(iv) séparation dudit complexe héparine-protéine constituant un variant de HGF d'avec ledit concentré ;

(v) traitement dudit complexe héparine-protéine constituant un variant de HGF dans des conditions telles que ladite protéine constituant un variant de HGF se dissocie de l'héparine de sorte qu'une solution de protéine constituant un variant de HGF libre est formée ; et

(vi) fractionnement de ladite solution par chromatographie d'exclusion de sorte que la protéine constituant un variant de HGF est séparée des composants restants.

20. Procédé de production d'une protéine constituant un variant de HGF à partir de cellules cultivées selon la revendication 16, où lesdites cellules produisant une protéine constituant un variant de HGF sont des cellules de léiomyosacrome SK-LMS-1 humaines.

21. Utilisation de la protéine constituant un variant de HGF selon l'une des revendications 1 à 8 pour la préparation d'une composition pour inhiber la croissance de cellules, ladite composition contenant une quantité thérapeutique de la protéine constituant un variant de HGF dans des conditions telles que la croissance des cellules est inhibée.

22. Procédé de diagnostic de troubles de la croissance comprenant les étapes de :

(i) isolement d'ARNm à partir d'un échantillon biologique,

(ii) mise en contact dudit ARNm avec un segment d'ADN selon l'une des revendications 9 à 14, et

(iii) détection de la présence d'hybrides ARN-ADN.

23. Procédé de diagnostic de troubles de la croissance par hybridation in situ comprenant les étapes de :

(i) mise en contact de l'ARNm contenu dans un échantillon isolé avec un segment d'ADN selon l'une des revendications 9 à 14 ou un fragment d'ARN dérivé de celui-ci, et

(ii) détection de la présence d'hybrides ARN-ADN.

24. Procédé in vitro d'inhibition de la croissance de cellules comprenant la mise en contact desdites cellules in vitro avec une quantité thérapeutique de la protéine constituant un variant de HGF selon l'une quelconque des revendications 1 à 8 dans des conditions telles que la croissance des cellules est inhibée.

## FIGURE 1

FIGURE 2

## FIGURE 3

NK2 Coding Sequence

```
                                    27                                    54
ATG TGG GTG ACC AAA CTC CTG CCA GCC CTG CTG CTG CAG CAT GTC CTC CTG CAT
MET Trp Val Thr Lys Leu Leu Pro Ala Leu Leu Leu Gln His Val Leu Leu His


                                    81                                   108
CTC CTC CTG CTC CCC ATC GCC ATC CCC TAT GCA GAG GGA CAA AGG AAA AGA AGA
Leu Leu Leu Leu Pro Ile Ala Ile Pro Tyr Ala Glu Gly Gln Arg Lys Arg Arg


                                   135                                   162
AAT ACA ATT CAT GAA TTC AAA AAA TCA GCA AAG ACT ACC CTA ATC AAA ATA GAT
Asn Thr Ile His Glu Phe Lys Lys Ser Ala Lys Thr Thr Leu Ile Lys Ile Asp


                                   189                                   216
CCA GCA CTG AAG ATA AAA ACC AAA AAA GTG AAT ACT GCA GAC CAA TGT GCT AAT
Pro Ala Leu Lys Ile Lys Thr Lys Lys Val Asn Thr Ala Asp Gln Cys Ala Asn


                                   243                                   270
AGA TGT ACT AGG AAT AAA GGA CTT CCA TTC ACT TGC AAG GCT TTT GTT TTT GAT
Arg Cys Thr Arg Asn Lys Gly Leu Pro Phe Thr Cys Lys Ala Phe Val Phe Asp


                                   297                                   324
AAA GCA AGA AAA CAA TGC CTC TGG TTC CCC TTC AAT AGC ATG TCA AGT GGA GTG
Lys Ala Arg Lys Gln Cys Leu Trp Phe Pro Phe Asn Ser MET Ser Ser Gly Val


                                   351                                   378
AAA AAA GAA TTT GGC CAT GAA TTT GAC CTC TAT GAA AAC AAA GAC TAC ATT AGA
Lys Lys Glu Phe Gly His Glu Phe Asp Leu Tyr Glu Asn Lys Asp Tyr Ile Arg


                                   405                                   432
AAC TGC ATC ATT GGT AAA GGA CGC AGC TAC AAG GGA ACA GTA TCT ATC ACT AAG
Asn Cys Ile Ile Gly Lys Gly Arg Ser Tyr Lys Gly Thr Val Ser Ile Thr Lys


                                   459                                   486
AGT GGC ATC AAA TGT CAG CCC TGG AGT TCC ATG ATA CCA CAC GAA CAC AGC TTT
Ser Gly Ile Lys Cys Gln Pro Trp Ser Ser MET Ile Pro His Glu His Ser Phe


                                   513                                   540
TTG CCT TCG AGC TAT CGG GGT AAA GAC CTA CAG GAA AAC TAC TGT CGA AAT CCT
Leu Pro Ser Ser Tyr Arg Gly Lys Asp Leu Gln Glu Asn Tyr Cys Arg Asn Pro


                                   567                                   594
CGA GGG GAA GAA GGG GGA CCC TGG TGT TTC ACA AGC AAT CCA GAG GTA CGC TAC
Arg Gly Glu Glu Gly Gly Pro Trp Cys Phe Thr Ser Asn Pro Glu Val Arg Tyr
```

EP 0 571 387 B1

## FIGURE 3 (CONT'D)

```
                                        621                                        648
GAA GTC TGT GAC ATT CCT CAG TGT TCA GAA GTT GAA TGC ATG ACC TGC AAT GGG
Glu Val Cys Asp Ile Pro Gln Cys Ser Glu Val Glu Cys Met Thr Cys Asn Gly


                                        675                                        702
GAG AGT TAT CGA GGT CTC ATG GAT CAT ACA GAA TCA GGC AAG ATT TGT CAG CGC
Glu Ser Tyr Arg Gly Leu Met Asp His Thr Glu Ser Gly Lys Ile Cys Gln Arg


                                        729                                        756
TGG GAT CAT CAG ACA CCA CAC CGG CAC AAA TTC TTG CCT GAA AGA TAT CCC GAC
Trp Asp His Gln Thr Pro His Arg His Lys Phe Leu Pro Glu Arg Tyr Pro Asp


                                        783                                        810
AAG GGC TTT GAT GAT AAT TAT TGC CGC AAT CCC GAT GGC CAG CCG AGG CCA TGG
Lys Gly Phe Asp Asp Asn Tyr Cys Arg Asn Pro Asp Gly Gln Pro Arg Pro Trp


                                        837                                        864
TGC TAT ACT CTT GAC CCT CAC ACC CGC TGG GAG TAC TGT GCA ATT AAA ACA TGC
Cys Tyr Thr Leu Asp Pro His Thr Arg Trp Glu Tyr Cys Ala Ile Lys Thr Cys


GAG ACA TAA
Glu Thr  .  .
```

25

FIGURE 4

A

```
         0        0.5        1.0        1.5       2.0 kb
         |    |    |    |    |    |    |    |    |    |

         |S| N | K1 | K2 | K3 | K4 |L| Protease-like |

         |S| N | K1 | K2 |

  P3             P1 (P1B)   P4      P2 (P2B)

              Lys Thr Cys Glu Thr End
              AAAACATGCGAGACATAACAT      EXON

              GTAAGTGA ~400 bp CTCCCCAG   INTRON
```

B

```
            1    2    3    4    5

  620 bp —

  220 bp —
```

FIGURE 5

## FIGURE 6

FIGURE 7

A

B

FIGURE 8

## FIGURE 9

### NK1 Coding Sequence

```
                                         27                                    54
ATG TGG GTC ACC AAA CTC CTG CCA GCC CTG CTG CTG CAG CAT GTC CTC CTG CAT
MET Trp Val Thr Lys Leu Leu Pro Ala Leu Leu Leu Gln His Val Leu Leu His


                                         81                                   108
CTC CTC CTG CTC CCC ATC GCC ATC CCC TAT GCA GAG GGA CAA AGG AAA AGA AGA
Leu Leu Leu Leu Pro Ile Ala Ile Pro Tyr Ala Glu Gly Gln Arg Lys Arg Arg


                                        135                                   162
AAT ACA ATT CAT GAA TTC AAA AAA TCA GCA AAG ACT ACC CTA ATC AAA ATA GAT
Asn Thr Ile His Glu Phe Lys Lys Ser Ala Lys Thr Thr Leu Ile Lys Ile Asp


                                        189                                   216
CCA GCA CTG AAG ATA AAA ACC AAA AAA GTC AAT ACT GCA GAC CAA TGT GCT AAT
Pro Ala Leu Lys Ile Lys Thr Lys Lys Val Asn Thr Ala Asp Gln Cys Ala Asn


                                        243                                   270
AGA TGT ACT AGG AAT AAA GGA CTT CCA TTC ACT TGC AAG GCT TTT GTT TTT GAT
Arg Cys Thr Arg Asn Lys Gly Leu Pro Phe Thr Cys Lys Ala Phe Val Phe Asp


                                        297                                   324
AAA GCA AGA AAA CAA TGC CTC TGG TTC CCC TTC AAT AGC ATG TCA AGT GGA GTG
Lys Ala Arg Lys Gln Cys Leu Trp Phe Pro Phe Asn Ser MET Ser Ser Gly Val


                                        351                                   378
AAA AAA GAA TTT GGC CAT GAA TTT GAC CTC TAT GAA AAC AAA GAC TAC ATT AGA
Lys Lys Glu Phe Gly His Glu Phe Asp Leu Tyr Glu Asn Lys Asp Tyr Ile Arg


                                        405                                   432
AAC TGC ATC ATT GGT AAA GGA CGC AGC TAC AAG GGA ACA GTA TCT ATC ACT AAG
Asn Cys Ile Ile Gly Lys Gly Arg Ser Tyr Lys Gly Thr Val Ser Ile Thr Lys


                                        459                                   486
AGT GGC ATC AAA TGT CAG CCC TGG AGT TCC ATG ATA CCA CAC GAA CAC AGC TTT
Ser Gly Ile Lys Cys Gln Pro Trp Ser Ser MET Ile Pro His Glu His Ser Phe


                                        513                                   540
TTG CCT TCG AGC TAT CGG GGT AAA GAC CTA CAG GAA AAC TAC TGT CGA AAT CCT
Leu Pro Ser Ser Tyr Arg Gly Lys Asp Leu Gln Glu Asn Tyr Cys Arg Asn Pro


                                        567                                   594
CGA GGG GAA GAA GGG GGA CCC TGG TGT TTC ACA AGC AAT CCA GAG GTA CGC TAC
Arg Gly Glu Glu Gly Gly Pro Trp Cys Phe Thr Ser Asn Pro Glu Val Arg Tyr
```

SEQUENCE LISTING

<110> The United States of America, as represented by the Secretary United States Department of Commerce

<120> A non-mitogenic competitive HGF antagonist

<130> 3J23940-3EP

<140> 91917603.2

<141> 1991-09-10

<150> PCT/US91/06368

<151> 1991-09-10

<150> US 655,502

<151> 1991-02-15

<150> US 582, 063

<151> 1990-09-14

<160> 4

<170> PatentIn Ver. 2.1

<210> 1

<211> 873

<212> DNA

<213> lung fibroblast cell line M426

<220>

<221> CDS

<222> (1)..(873)

<400> 1

```
atg tgg gtg acc aaa ctc ctg cca gcc ctg ctg ctg cag cat gtc ctc    48
Met Trp Val Thr Lys Leu Leu Pro Ala Leu Leu Leu Gln His Val Leu
 1               5              10                15

ctg cat ctc ctc ctg ctc ccc atc gcc atc ccc tat gca gag gga caa    96
Leu His Leu Leu Leu Leu Pro Ile Ala Ile Pro Tyr Ala Glu Gly Gln
            20              25              30

agg aaa aga aga aat aca att cat gaa ttc aaa aaa tca gca aag act   144
Arg Lys Arg Arg Asn Thr Ile His Glu Phe Lys Lys Ser Ala Lys Thr
        35              40              45

acc cta atc aaa ata gat cca gca ctg aag ata aaa acc aaa aaa gtg   192
Thr Leu Ile Lys Ile Asp Pro Ala Leu Lys Ile Lys Thr Lys Lys Val
     50              55              60

aat act gca gac caa tgt gct aat aga tgt act agg aat aaa gga ctt   240
Asn Thr Ala Asp Gln Cys Ala Asn Arg Cys Thr Arg Asn Lys Gly Leu
 65              70              75              80

cca ttc act tgc aag gct ttt gtt ttt gat aaa gca aga aaa caa tgc   288
Pro Phe Thr Cys Lys Ala Phe Val Phe Asp Lys Ala Arg Lys Gln Cys
                85              90              95

ctc tgg ttc ccc ttc aat agc atg tca agt gga gtg aaa aaa gaa ttt   336
Leu Trp Phe Pro Phe Asn Ser Met Ser Ser Gly Val Lys Lys Glu Phe
                100             105             110
```

```
ggc cat gaa ttt gac ctc tat gaa aac aaa gac tac att aga aac tgc   384
Gly His Glu Phe Asp Leu Tyr Glu Asn Lys Asp Tyr Ile Arg Asn Cys
        115                 120                 125

atc att ggt aaa gga cgc agc tac aag gga aca gta tct atc act aag   432
Ile Ile Gly Lys Gly Arg Ser Tyr Lys Gly Thr Val Ser Ile Thr Lys
    130                 135                 140

agt ggc atc aaa tgt cag ccc tgg agt tcc atg ata cca cac gaa cac   480
Ser Gly Ile Lys Cys Gln Pro Trp Ser Ser Met Ile Pro His Glu His
145                 150                 155                 160

agc ttt ttg cct tcg agc tat cgg ggt aaa gac cta cag gaa aac tac   528
Ser Phe Leu Pro Ser Ser Tyr Arg Gly Lys Asp Leu Gln Glu Asn Tyr
                165                 170                 175

tgt cga aat cct cga ggg gaa gaa ggg gga ccc tgg tgt ttc aca agc   576
Cys Arg Asn Pro Arg Gly Glu Glu Gly Gly Pro Trp Cys Phe Thr Ser
            180                 185                 190

aat cca gag gta cgc tac gaa gtc tgt gac att cct cag tgt tca gaa   624
Asn Pro Glu Val Arg Tyr Glu Val Cys Asp Ile Pro Gln Cys Ser Glu
            195                 200                 205

gtt gaa tgc atg acc tgc aat ggg gag agt tat cga ggt ctc atg gat   672
Val Glu Cys Met Thr Cys Asn Gly Glu Ser Tyr Arg Gly Leu Met Asp
    210                 215                 220

cat aca gaa tca ggc aag att tgt cag cgc tgg gat cat cag aca cca   720
His Thr Glu Ser Gly Lys Ile Cys Gln Arg Trp Asp His Gln Thr Pro
225                 230                 235                 240

cac cgg cac aaa ttc ttg cct gaa aga tat ccc gac aag ggc ttt gat   768
His Arg His Lys Phe Leu Pro Glu Arg Tyr Pro Asp Lys Gly Phe Asp
                245                 250                 255

gat aat tat tgc cgc aat ccc gat ggc cag ccg agg cca tgg tgc tat   816
Asp Asn Tyr Cys Arg Asn Pro Asp Gly Gln Pro Arg Pro Trp Cys Tyr
                260                 265                 270

act ctt gac cct cac acc cgc tgg gag tac tgt gca att aaa aca tgc   864
Thr Leu Asp Pro His Thr Arg Trp Glu Tyr Cys Ala Ile Lys Thr Cys
        275                 280                 285

gag aca taa                                                        873
Glu Thr
    290
```

<210> 2

<211> 290

<212> PRT

<213> lung fibroblast cell line M426

<400> 2

```
Met Trp Val Thr Lys Leu Leu Pro Ala Leu Leu Leu Gln His Val Leu
1             5                 10                      15

Leu His Leu Leu Leu Leu Pro Ile Ala Ile Pro Tyr Ala Glu Gly Gln
            20              25              30

Arg Lys Arg Arg Asn Thr Ile His Glu Phe Lys Lys Ser Ala Lys Thr
        35              40                  45

Thr Leu Ile Lys Ile Asp Pro Ala Leu Lys Ile Lys Thr Lys Lys Val
    50              55              60

Asn Thr Ala Asp Gln Cys Ala Asn Arg Cys Thr Arg Asn Lys Gly Leu
    65              70              75                  80

Pro Phe Thr Cys Lys Ala Phe Val Phe Asp Lys Ala Arg Lys Gln Cys
                85              90                  95

Leu Trp Phe Pro Phe Asn Ser Met Ser Ser Gly Val Lys Lys Glu Phe
            100             105             110

Gly His Glu Phe Asp Leu Tyr Glu Asn Lys Asp Tyr Ile Arg Asn Cys
        115             120             125

Ile Ile Gly Lys Gly Arg Ser Tyr Lys Gly Thr Val Ser Ile Thr Lys
    130             135             140

Ser Gly Ile Lys Cys Gln Pro Trp Ser Ser Met Ile Pro His Glu His
145             150             155             160

Ser Phe Leu Pro Ser Ser Tyr Arg Gly Lys Asp Leu Gln Glu Asn Tyr
            165             170             175

Cys Arg Asn Pro Arg Gly Glu Glu Gly Gly Pro Trp Cys Phe Thr Ser
            180             185             190

Asn Pro Glu Val Arg Tyr Glu Val Cys Asp Ile Pro Gln Cys Ser Glu
        195             200             205

Val Glu Cys Met Thr Cys Asn Gly Glu Ser Tyr Arg Gly Leu Met Asp
    210             215             220

His Thr Glu Ser Gly Lys Ile Cys Gln Arg Trp Asp His Gln Thr Pro
225             230             235             240

His Arg His Lys Phe Leu Pro Glu Arg Tyr Pro Asp Lys Gly Phe Asp
            245             250             255

Asp Asn Tyr Cys Arg Asn Pro Asp Gly Gln Pro Arg Pro Trp Cys Tyr
            260             265             270

Thr Leu Asp Pro His Thr Arg Trp Glu Tyr Cys Ala Ile Lys Thr Cys
            275             280             285

Glu Thr
290
```

<210> 3

<211> 594

<212> DNA

<213> lung fibroblast cell line M426

<220>

<221> CDS

<222> (1)..(594)

<400> 3

```
atg tgg gtg acc aaa ctc ctg cca gcc ctg ctg ctg cag cat gtc ctc        48
Met Trp Val Thr Lys Leu Leu Pro Ala Leu Leu Leu Gln His Val Leu
 1               5                  10                  15

ctg cat ctc ctc ctg ctc ccc atc gcc atc ccc tat gca gag gga caa        96
Leu His Leu Leu Leu Leu Pro Ile Ala Ile Pro Tyr Ala Glu Gly Gln
                 20                  25                  30

agg aaa aga aga aat aca att cat gaa ttc aaa aaa tca gca aag act       144
Arg Lys Arg Arg Asn Thr Ile His Glu Phe Lys Lys Ser Ala Lys Thr
             35                  40                  45

acc cta atc aaa ata gat cca gca ctg aag ata aaa acc aaa aaa gtg       192
Thr Leu Ile Lys Ile Asp Pro Ala Leu Lys Ile Lys Thr Lys Lys Val
         50                  55                  60

aat act gca gac caa tgt gct aat aga tgt act agg aat aaa gga ctt       240
Asn Thr Ala Asp Gln Cys Ala Asn Arg Cys Thr Arg Asn Lys Gly Leu
 65                  70                  75                  80

cca ttc act tgc aag gct ttt gtt ttt gat aaa gca aga aaa caa tgc       288
Pro Phe Thr Cys Lys Ala Phe Val Phe Asp Lys Ala Arg Lys Gln Cys
                 85                  90                  95

ctc tgg ttc ccc ttc aat agc atg tca agt gga gtg aaa aaa gaa ttt       336
Leu Trp Phe Pro Phe Asn Ser Met Ser Ser Gly Val Lys Lys Glu Phe
                100                 105                 110

ggc cat gaa ttt gac ctc tat gaa aac aaa gac tac att aga aac tgc       384
Gly His Glu Phe Asp Leu Tyr Glu Asn Lys Asp Tyr Ile Arg Asn Cys
                115                 120                 125

atc att ggt aaa gga cgc agc tac aag gga aca gta tct atc act aag       432
Ile Ile Gly Lys Gly Arg Ser Tyr Lys Gly Thr Val Ser Ile Thr Lys
        130                 135                 140

agt ggc atc aaa tgt cag ccc tgg agt tcc atg ata cca cac gaa cac       480
Ser Gly Ile Lys Cys Gln Pro Trp Ser Ser Met Ile Pro His Glu His
145                 150                 155                 160

agc ttt ttg cct tcg agc tat cgg ggt aaa gac cta cag gaa aac tac       528
Ser Phe Leu Pro Ser Ser Tyr Arg Gly Lys Asp Leu Gln Glu Asn Tyr
                165                 170                 175

tgt cga aat cct cga ggg gaa gaa ggg gga ccc tgg tgt ttc aca agc       576
Cys Arg Asn Pro Arg Gly Glu Glu Gly Gly Pro Trp Cys Phe Thr Ser
                180                 185                 190

aat cca gag gta cgc tac  594
Asn Pro Glu Val Arg Tyr
                195
```

<210> 4

<211> 198

<212> PRT

<213> lung fibroblast cell line M426

15

<400> 4

```
Met Trp Val Thr Lys Leu Leu Pro Ala Leu Leu Leu Gln His Val Leu
 1               5                  10              15

Leu His Leu Leu Leu Leu Pro Ile Ala Ile Pro Tyr Ala Glu Gly Gln
          20                  25              30

Arg Lys Arg Arg Asn Thr Ile His Glu Phe Lys Lys Ser Ala Lys Thr
         35                  40              45

Thr Leu Ile Lys Ile Asp Pro Ala Leu Lys Ile Lys Thr Lys Lys Val
     50                  55              60

Asn Thr Ala Asp Gln Cys Ala Asn Arg Cys Thr Arg Asn Lys Gly Leu
 65                  70              75                  80

Pro Phe Thr Cys Lys Ala Phe Val Phe Asp Lys Ala Arg Lys Gln Cys
             85                  90                  95

Leu Trp Phe Pro Phe Asn Ser Met Ser Ser Gly Val Lys Lys Glu Phe
             100                 105                 110

Gly His Glu Phe Asp Leu Tyr Glu Asn Lys Asp Tyr Ile Arg Asn Cys
         115                 120                 125

Ile Ile Gly Lys Gly Arg Ser Tyr Lys Gly Thr Val Ser Ile Thr Lys
     130                 135                 140

Ser Gly Ile Lys Cys Gln Pro Trp Ser Ser Met Ile Pro His Glu His
 145                 150                 155                 160

Ser Phe Leu Pro Ser Ser Tyr Arg Gly Lys Asp Leu Gln Glu Asn Tyr
             165                 170                 175

Cys Arg Asn Pro Arg Gly Glu Glu Gly Gly Pro Trp Cys Phe Thr Ser
             180                 185                 190

Asn Pro Glu Val Arg Tyr
             195
```